# EUROPÄISCHE PATENTANMELDUNG

(11) **EP 3 808 341 A1**
(43) Veröffentlichungstag der Anmeldung: **21.04.2021**
(21) Anmeldenummer: 19203580.6
(22) Anmeldetag: 16.10.2019
(51) Int. Cl.: A61K 9/16, A61K 9/20, A61K 31/05, A61K 31/352

(54) **KONTROLLIERT FREISETZENDE FORMULIERUNGEN STARK LIPOPHILER PHYSIOLOGISCH AKTIVER SUBSTANZEN**

(71) Anmelder: ADD Advanced Drug Delivery Technologies, Ltd., 4133 Pratteln (CH)
(72) Erfinder: Nowak, Mirko, 79540 Lörrach (DE); Nowak, Jesko Jay, 79540 Lörrach (DE); Grave, Annette, 79541 Lörrach (DE); Wentzlaff, Monika, 79540 Lörrach (DE); Barthold, Sarah, 88444 Ummendorf (DE); Geugelin, Christian, 79588 Efringen-Kirchen (DE)
(74) Vertreter: Breuer, Markus

(57) **Zusammenfassung**

Es wird eine feste Darreichungsform bereitgestellt, die eine Matrix mit einer oder mehreren stark lipophilen physiologisch aktiven Substanzen, einem oder mehreren wasserlöslichen Bindemitteln und nicht mehr als 20 Gew.-%, bezogen auf das Gewicht aller Komponenten, an weiteren Hilfsstoffen aufweist.

## Beschreibung

### Gebiet der Erfindung

Die Erfindung betrifft Formulierungen für stark lipophile physiologisch aktive Substanzen, insbesondere kontrolliert freisetzende Formulierungen, sowie deren Herstellung. Bei den stark lipophilen physiologisch aktiven Substanzen handelt es sich beispielsweise um pharmazeutische Wirkstoffe. Ein Beispiel für stark lipophile pharmazeutische Wirkstoffe sind Cannabinoide.

### Hintergrund der Erfindung

Eine Reihe von physiologisch aktiven Substanzen haben stark lipophile Eigenschaften, d.h., sie weisen einen relativ hohen log P auf, beispielsweise einen log P von 4 oder mehr auf, wobei der log P der dekadische Logarithmus des n-Octanol/Wasser-Verteilungskoeffizienten ist.

Die Bereitstellung von Formulierungen, insbesondere oralen Formulierungen, mit solchen physiologisch aktiven Substanzen stellt eine besondere Herausforderung dar, insbesondere wenn eine kontrollierte Freisetzung der physiologisch aktiven Substanzen erzielt werden soll.

Zu den physiologisch aktiven Substanzen mit stark lipophilen Eigenschaften gehören Cannabinoide.

Cannabinoide sind eine heterogene Gruppe pharmakologisch aktiver Substanzen, die eine Affinität zu den sogenannten Cannabinoid-Rezeptoren aufweisen. Zu den Cannabinoiden gehören beispielsweise Tetrahydrocannabinol (THC) und das nicht psychoaktive Cannabidiol (CBD).

Cannabinoide haben erhebliches Interesse als Arzneistoffe gefunden. So gibt es Hinweise darauf, dass die Verabreichung von Cannabinoiden bei einer Reihe von klinischen Zuständen von Vorteil sein kann, darunter Schmerzen, Entzündungen, Epilepsie, Schlafstörungen, Symptome von Multipler Sklerose, Anorexie und Schizophrenie (N. Bruni et al., Cannabinoid Delivery Systems for Pain and Inflammation Treatment. Molecules 2018, 23, 2478).

Die Bereitstellung geeigneter Darreichungsformen ist allerdings mit Schwierigkeiten verbunden, da Cannabinoide stark lipophile Moleküle (log P 6-7) mit sehr geringer Wasserlöslichkeit (2-10 µg / ml) sind.

So hat die geringe orale Bioverfügbarkeit von Cannabinoiden dazu geführt, dass transdermale, intranasale und transmukosale Verabreichung vorgeschlagen wurden.

Daneben gehören aufgrund der hohen Lipophilie von Cannabinoiden Salzbildung (d.h. pH-Einstellung), Cosolvenz (z. B. Ethanol, Propylenglykol, PEG400), Mizellisierung (z. B. Polysorbat 80, Cremophor-ELP), Emulgierung einschließlich Mikro- und Nano-Emulgierung, Komplexierung (z. B. Cyclodextrine) und Einkapselung in Formulierungen auf Lipidbasis (z. B. Liposomen) zu den Formulierungsstrategien, die im Stand der Technik in Betracht gezogen wurden. Zudem wurden Nanopartikel-Systeme vorgeschlagen (N. Bruni et al., a.a.O.).

In der Patentliteratur sind verschiedene feste orale Darreichungsformen vorgeschlagen worden, so in WO 2008/024490 A2 und in WO 2018/035030 A1. Diese Dokumente enthalten keine Daten zum Freisetzungsverhalten, so dass die praktische Eignung der vorgeschlagenen Formen für die Verabreichung von Cannabinoiden unklar bleibt.

WO 2015/065179 A1 beschreibt komprimierte Tabletten, die neben Cannabidiol Lactose und Saccharosefettsäuremonoester enthalten.

Dronabinol (Δ9-THC) wird in Form von Kapseln (Marinol®) und als orale Lösung (Syndros®) vermarktet. Bei den Marinol®-Kapseln handelt es sich um Weichgelatinekapseln, die den Wirkstoff in Sesamöl enthalten.

Das Nabiximols-haltige Fertigarzneimittel Sativex® ist ein Mundspray, das auf die Innenseite der Wange gesprüht wird.

Das unlängst zugelassene Präparat Epidiolex zur Behandlung bestimmter Formen der Epilepsie wird in Form einer oralen Lösung zur Verfügung gestellt, die neben dem Wirkstoff Cannabidiol die Hilfsstoffe absolutes Ethanol, Sesamöl, Erdbeeraroma und Sucralose enthält.

Ungeachtet all dieser Vorschläge besteht aber weiterhin ein Bedarf an verbesserten Darreichungsformen für stark lipophile physiologisch aktive Substanzen, beispielsweise pharmazeutische Wirkstoffe wie Cannabinoide, insbesondere an oralen festen Darreichungsformen.

### Aufgaben und Kurzdarstellung der Erfindung

Eine Aufgabe der Erfindung besteht darin, feste Darreichungsformen, insbesondere orale feste Darreichungsformen, für stark lipophile physiologisch aktive Substanzen, wie Cannabinoide, bereitzustellen, die die physiologisch aktive Substanz/die physiologisch aktiven Substanzen freisetzen und die in einfacher Weise hergestellt werden können.

Diese Aufgabe wird durch die Bereitstellung einer festen Darreichungsform gelöst, die eine Matrix mit einer oder mehreren stark lipophilen physiologisch aktiven Substanzen; einem oder mehreren wasserlöslichen Bindemitteln und nicht mehr als 20 Gew.-%, bezogen auf das Gewicht aller Komponenten, an weiteren Hilfsstoffen aufweist, wobei eine physiologisch aktive Substanz stark lipophil ist, wenn sie einen log P von 4 oder mehr hat.

Überraschend wurde festgestellt, dass sich so feste Darreichungsformen, insbesondere orale feste Darreichungsformen, von stark lipophilen physiologisch aktiven Substanzen bereitstellen lassen, wobei über die Menge an wasserlöslichen Bindemittel(n), bezogen auf die Menge an stark lipophiler Substanz/stark lipophilen Substanzen, die Freisetzung gesteuert werden kann. Der Einsatz eines oder mehrerer wasserlöslicher Bindemittel erlaubt nicht nur die Ausbildung einer Matrix mit der physiologisch aktiven Substanz/den physiologisch aktiven Substanzen, sondern dient auch der Steuerung der Freisetzung. Insbesondere fördert ein wasserlösliches Bindemittel die Freisetzung der nur sehr wenig in Wasser löslichen stark lipophilen Substanzen. Erst durch das Bindemittel werden diese in ausreichender Menge und Geschwindigkeit freigesetzt.

Weitere Aufgaben und deren Lösung ergeben sich aus der nachstehenden ausführlichen Darstellung der Erfindung.

### Kurze Beschreibung der Figuren

Die Erfindung wird nachstehend unter Bezugnahme auf die Figur näher erläutert.

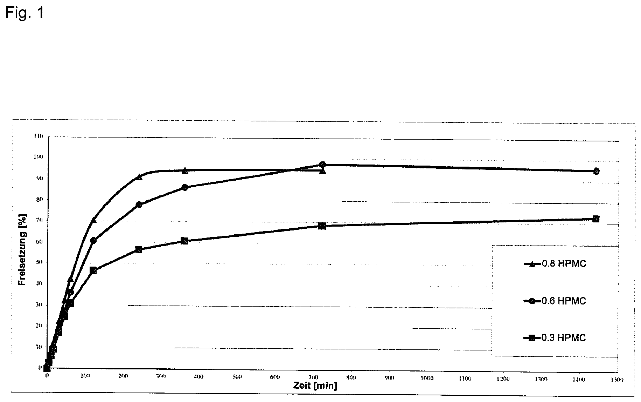

Fig. 1 zeigt die in vitro-Freisetzung aus drei Pelletprodukten, die 2-[1R-3-Methyl-6R-(1-methylethenyl)-2-cyclohexen-1-yl]-5-pentyl-1,3-benzenediol als Wirkstoff und niedrigviskose Hydroxypropylmethylcellulose enthalten.

### Ausführliche Darstellung der Erfindung

Die erfindungsgemäß bereitgestellten Darreichungsformen enthalten eine oder mehrere stark lipophile physiologisch aktive Substanzen.

Eine Substanz ist stark lipophil, wenn sie einen log P von 4 oder mehr aufweist. Der log P ist der dekadische Logarithmus des n-Octanol/Wasser-Verteilungskoeffizienten. Der Verteilungskoeffizient kann experimentell ermittelt werden. Werte beziehen sich typischerweise auf Raumtemperatur (25°C). Der Verteilungskoeffizient kann auch näherungsweise aus der Molekülstruktur errechnet werden.

Die erfindungsgemäßen Pellets eignen sich besonders für physiologisch aktive Substanzen mit einem log P von 5 oder mehr und ganz besonders für solche mit einem log P von 6 oder mehr.

Der Begriff "physiologisch aktive Substanz" bezieht sich auf eine Substanz, die einem Menschen oder einem Tier verabreicht wird, um eine Wirkung im menschlichen oder tierischen Körper zu entfalten. Die physiologisch aktive Substanz kann beispielsweise ein pharmazeutischer Wirkstoff eines Human- oder Tierarzneimittels oder ein Nahrungsergänzungsmittel sein.

Ein Beispiel für stark lipophile pharmazeutische Wirkstoffe, die erfindungsgemäß verwendet werden können, sind Cannabinoide.

Bei den Cannabinoiden kann es sich sowohl um Phytocannabinoide als auch um synthetische Cannabinoide handeln.

Die Phytocannabinoide sind eine Gruppe von etwa 70 terpenophenolischen Verbindungen (V.R. Preedy (Hrsg.), Handbook of Cannabis and Related Pathologies (1997)). Diese Verbindungen enthalten typischerweise einen Monoterpenrest, der an einen Phenolring gebunden ist und eine C₃-C₅-Alkylkette aufweist, die sich in der meta-Position zur phenolischen Hydroxylgruppe befindet.

Eine bevorzugte Gruppe von Cannabinoiden sind Tetrahydrocannabinole der folgenden allgemeinen Formel (1): 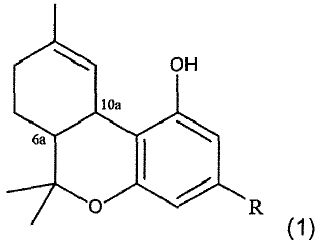 worin R ausgewählt ist aus C₁-C₂₀-Alkyl, C₂-C₂₀-Alkenyl oder C₂-C₂₀-Alkinyl und optional einen oder mehrere Substituenten aufweist.

In einer weiter bevorzugten Gruppe von Verbindungen der vorstehenden allgemeinen Formel (1) ist R ausgewählt aus C₁-C₁₀-Alkyl oder C₂-C₁₀-Alkenyl und weist optional einen oder mehrere Substituenten auf.

Insbesondere ist R in der Formel (1) ein Alkylrest der Formel C₅H₁₁.

Verbindungen der allgemeinen Formel (1) können in Form von Stereoisomeren vorliegen. Vorzugsweise weisen die Zentren 6a und 10a jeweils die R-Konfiguration auf. Bei dem Tetrahydrocannabinol handelt es sich insbesondere um Δ9-THC mit dem chemischen Namen (6aR,10aR)-6,6,9-Trimethyl-3-pentyl-6a,7,8,10a-tetrahydro-6H-benzo[c]chromen-1-ol. Die Struktur wird durch die nachstehende Formel (2) wiedergegeben:

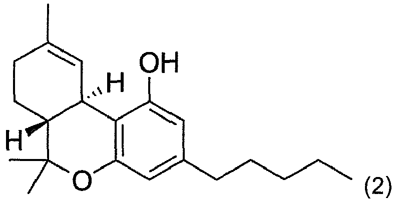

Eine weitere bevorzugte Gruppe von Cannabinoiden sind Cannabidiole der folgenden allgemeinen Formel (3): 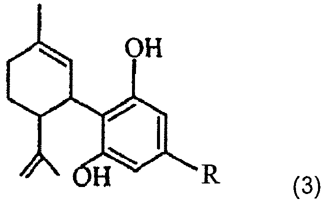 worin R ausgewählt ist aus C₁-C₂₀-Alkyl, C₂-C₂₀-Alkenyl oder C₂-C₂₀-Alkinyl und optional einen oder mehrere Substituenten aufweist.

In einer weiter bevorzugten Gruppe von Verbindungen der vorstehenden allgemeinen Formel (3) ist R ausgewählt aus C₁-C₁₀-Alkyl oder C₂-C₁₀-Alkenyl und weist optional einen oder mehrere Substituenten auf.

Insbesondere ist R in der Formel (3) ein Alkylrest der Formel C₅H₁₁.

Bei dem Cannabidiol handelt es sich insbesondere um 2-[1R-3-Methyl-6R-(1-methylethenyl)-2-cyclohexen-1-yl]-5-pentyl-1,3-benzenediol.

Erfindungsgemäß kann als Wirkstoff auch eine Kombination aus um Δ9-THC ((6aR,10aR)-6,6,9-Trimethyl-3-pentyl-6a,7,8,10a-tetrahydro-6H-benzo[c]chromen-1-ol) und CBD (2-[1R-3-Methyl-6R-(1-methylethenyl)-2-cyclohexen-1-yl]-5-pentyl-1,3-benzenediol) verwendet werden.

Eine weitere bevorzugte Gruppe von Cannabinoiden sind Cannabinole der folgenden allgemeinen Formel (4): 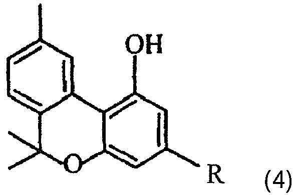 worin R ausgewählt ist aus C₁-C₂₀-Alkyl, C₂-C₂₀-Alkenyl oder C₂-C₂₀-Alkinyl und optional einen oder mehrere Substituenten aufweist.

In einer weiter bevorzugten Gruppe von Verbindungen der vorstehenden allgemeinen Formel (4) ist R ausgewählt aus C₁-C₁₀-Alkyl oder C₂-C₁₀-Alkenyl und weist optional einen oder mehrere Substituenten auf.

Insbesondere ist R in der Formel (4) ein Alkylrest der Formel C₅H₁₁.

Bei dem Cannabinol handelt es sich insbesondere um 6,6,9-Trimethyl-3-pentyl-6*H-*dibenzo[*b*,*d*]pyran-1-ol.

Erfindungsgemäß können auch Cannabinoide oder Cannabinoid-Gemische aus Hanfextrakten verwendet werden.

Beispielsweise ist Nabiximols eine als Arzneistoff verwendete Pflanzenextraktmischung aus den Blättern und Blüten der Hanfpflanze (Cannabis sativa L.) mit standardisierten Gehalten an Tetrahydrocannabinol (THC) und Cannabidiol (CBD).

Es können auch synthetische Cannabinoide verwendet werden.

Dazu gehört 3-(1,1-Dimethylheptyl)-6,6a,7,8,10,10a-hexahydro-1-hydroxy-6,6-dimethyl-9*H*-dibenzo[*b*,*d*]pyran-9-on. Diese Verbindung enthält zwei stereogene Zentren. Der Arzneistoff Nabilon ist ein 1:1-Gemisch (Racemat) aus der (6a*R*,10a*R*)-Form und der (6aS,10aS)-Form. Nabilon ist ein erfindungsgemäß bevorzugtes Cannabinoid.

Ein weiteres Beispiel für ein synthetisches Cannabinoid ist JWH-018 (1-Naphthyl-(1-pentylindol-3-yl)methanon.

Eine oder mehrere stark lipophile physiologisch aktive Substanzen, wie ein oder mehrere pharmazeutische Wirkstoffe, wie Cannabinoide, sind erfindungsgemäß in einer Matrix enthalten. Die Matrix enthält vorzugsweise keine weiteren physiologisch aktiven Substanzen.

Die Matrix enthält ein oder mehrere wasserlösliche Bindemittel. Bei diesen Bindemitteln handelt es sich um polymere filmbildende Substanzen.

Beispiele für geeignete wasserlösliche Bindemittel sind Methylcellulose (MC), Hydroxypropylmethylcellulose (HPMC), Hydroxypropylcellulose (HPC), Hydroxyethylcellulose (HEC), Natrium-Carboxymethylcellulose (Na-CMC) und Polyvinylpyrrolidon (PVP).

Bevorzugt ist Hydroxypropylmethylcellulose (HPMC), insbesondere niedrigviskose HPMC, wie HPMC mit einer Viskosität einer 2% (Gew./Gew.) wässrigen Lösung bei 20°C von 6 mPa·s oder weniger.

Eine HPMC mit einer Viskosität einer 2% (Gew./Gew.) wässrigen Lösung bei 20°C von 3 mPa·s, wie sie unter der Handelsbezeichnung Pharmacoat® 603 verfügbar ist, ist besonders bevorzugt.

Die Matrix aus einer oder mehreren stark lipophilen physiologisch aktiven Substanzen und einem oder mehreren wasserlöslichen Bindemitteln kann weitere übliche Hilfsstoffe, wie einen oder mehrere Füll- oder Trägerstoffe, enthalten. Die Menge der weiteren Hilfsstoffe ist erfindungsgemäß auf nicht mehr als 20 Gew.-%, bezogen auf das Gewicht aller Komponenten, begrenzt. Vorzugsweise sind nicht mehr als 10 Gew.-%, bezogen auf das Gewicht aller Komponenten, an weiteren Hilfsstoffen enthalten.

In einer besonders bevorzugten Ausführungsform besteht die Matrix aus stark lipophiler physiologisch aktiver Substanz/stark lipophilen physiologisch aktiven Substanzen und Bindemittel(n), beispielsweise aus Cannabinoid(en) und Bindemittel(n).

Die Matrix enthält ein oder mehrere wasserlösliche Bindemittel, bezogen auf die Gesamtmenge an stark lipophilen physiologisch aktiven Substanzen, in einem Gesamtanteil von 0,1 - 10 Gew.-%, vorzugsweise in einem Gesamtanteil von 0,5 - 8 Gew.-% und insbesondere in einem Gesamtanteil von 1 - 6 Gew.-% enthält.

Es wird angenommen, dass bei einer zu geringen Menge an wasserlöslichem Bindemittel die Freisetzung nur sehr langsam und unvollständig erfolgt. Durch Wahl des Anteils in den angegebenen Bereichen kann die Freisetzung der physiologisch aktiven Substanz eingestellt werden. Beispielsweise kann die Freisetzung aus einer oralen Darreichungsform so eingestellt werden, dass die physiologisch aktive Substanz über die übliche Zeit der Magen-Darm-Passage abgegeben wird.

Die erfindungsgemäße feste orale Darreichungsform, die eine Matrix mit einer oder mehreren stark lipophilen physiologisch aktiven Substanzen aufweist, kann in beliebiger Form bereitgestellt und verwendet werden. Beispielsweise kann die Darreichungsform in Form eines Granulats, von Matrixpellets oder von Matrixtabletten bereitgestellt werden oder eine dieser Formen enthalten.

Die Herstellung kann auf an sich bekannte Weise erfolgen.

In einer bevorzugten Ausführungsform enthält die Darreichungsform Matrixpellets.

Die Matrixpellets haben typischerweise eine Größe im Bereich von 30 bis 1800 µm, wobei die Größe durch Siebanalyse bestimmt werden kann.

Die Matrixellets können als orale Darreichungsform verwendet werden, wobei sie z.B. in Sachets angeboten werden, oder sie können weiterverarbeitet werden.

Beispielsweise können die Matrixpellets mit einem oder mehreren weiteren Überzügen versehen werden. Damit ist eine zusätzliche Steuerung der Freisetzung möglich.

In einer bevorzugten Ausführungsform ist kein Überzug vorgesehen, der die Freisetzung steuert.

Die Matrixpellets können auch verwendet werden, um multipartikuläre Darreichungsformen zu erhalten. Sie können in Kapseln gefüllt oder in Tabletten eingearbeitet werden.

Matrixpellets mit unterschiedlichen Freisetzungsprofilen können in einer Darreichungsform (Kapsel/Tablette/Sachet) kombiniert werden.

Die erfindungsgemäßen oralen Darreichungsformen setzen die enthaltene stark lipophile physiologisch aktive Substanz oder, falls mehr als eine stark lipophile physiologisch aktive Substanz enthalten ist, sämtliche enthaltenen stark lipophilen physiologisch aktiven Substanzen nach Einnahme im Verdauungstrakt frei. Die Darreichungsformen dienen insbesondere der kontrollierten Freisetzung. Insbesondere setzen sie mehr als 30 Gew.-% und weniger als 80 Gew.-% der enthaltenen physiologisch aktiven Substanz innerhalb von zwei Stunden frei. Weiterhin setzen sie insbesondere mehr als 40 Gew.-% und weniger als 90 Gew.-% der enthaltenen physiologisch aktiven Substanz innerhalb von drei Stunden frei. Darüber hinaus setzen sie mehr als 50 Gew.-% und weniger als 95 Gew.-% der enthaltenen physiologisch aktiven Substanz innerhalb von vier Stunden frei. Falls mehr als eine physiologisch aktive Substanz enthalten ist, beziehen sich die Angaben auf sämtliche enthaltenen Substanzen.

Die Bestimmung der Freisetzung erfolgt dabei jeweils in einer Blattrührer-Apparatur in 1000 ml Phosphatpuffer pH 6.8 mit einem Zusatz an 0.4 % Tween® 80 bei 37° C.

### Beispiele

Die nachstehenden Beispiele zeigen, wie durch Einsatz einer wasserlöslichen filmbildenden Substanz die Freisetzung einer stark lipophilen physiologisch aktiven Substanz gesteuert werden kann.

### Beispiel 1 - Herstellung von Pellets

Es wurden Pellets unter Verwendung der in der nachstehenden Tabelle 1 angegebenen Mengen an Inhaltsstoffen hergestellt.

Dazu wurde 2-[1R-3-Methyl-6R-(1-methylethenyl)-2-cyclohexen-1-yl]-5-pentyl-1,3-benzenediol (Canapure PH) in Ethanol 96% gelöst. Dieser Wirkstoff weist einen log P von etwa 6,1 auf.

Eine weitere Lösung wurde hergestellt, indem HPMC (Pharmacoat® 603) in Wasser gelöst wurde.

Anschließend wurde die HPMC-Lösung schrittweise zu der Cannabidiol-Lösung gegeben.

Dann wurde amorphes Siliciumdioxid (Syloid® 244 FP) zugefügt.

Es wurde jeweils mit einem Propellerrüher gerührt.

Die erhaltene Sprühflüssigkeit wurde auf Starterkerne aus mikrokristalliner Cellulose (Cellets® 500) aufgesprüht.

Dies erfolgte in einer Wirbelschichtanlage vom Typ Mini-Glatt mit einem Wurster-Einsatz. Dabei betrug die Lufteinlasslufttemperatur 40°C. Die mittlere Sprührate betrug 0,5 g/min.

**Tabelle 1 - Eingesetzte Stoffe und Mengen**

| **Ansatz** | **HPMC 0.8** | **HPMC 0.6** | **HPMC 0.3** |
|---|---|---|---|
| **Feststoffe** | Menge | Menge | Menge |
| Cellets 500 | 60,01 g / 81,5 % | 60,00 g / 72,7 % | 60,00 g / 72,7 % |
| Canapure PH | 21,02 g / 16,1 % | 21,00 g / 24,2 % | 21,26 g / 24,5 % |
| Pharmacoat 603 | 1,05 g / 0,8 % | 0,53 g / 0,6 % | 0,26 g / 0,3 % |
| Syloid 244 FP | 2,10 g / 1,6 % | 2,10 g / 2,4% | 2,10 g / 2,4% |

| **Flüssigkeiten (nicht im Produkt enthalten)** | | | |
|---|---|---|---|
| Ethanol 96% | 79,81 g | 79,83 g | 79,82 g |
| Reinwasser | 25,20 g | 25,21 g | 25,21 g |

| **Sprühflüssigkeit** | | | |
|---|---|---|---|
| Feststoffanteil (Gew./Gew.) | 18,71 % | 18,36 % | 18,36 % |
| Versprühte Menge | 72,80 g | 122,50 g | 122,50 g |

**Tabelle 2 - Produkte**

| **Ansatz** | **HPMC 0.8** | **HPMC 0.6** | **HPMC 0.3** |
|---|---|---|---|
| Theoretische Ausbeute | 73,63 g | 82,49 g | 82,49 g |
| Praktische Ausbeute | 64,30 g / 87,33 % | 75,03 g / 90,95 % | 74,24 g / 90,00 % |
| Überzugsgewichtszunahme | 31,49 % | 66,82 % | 63,31 % |

### Beispiel 2 - Freisetzung

Die Freisetzung aus den in Beispiel 1 erhaltenen Pelletprodukten wird unter Verwendung einer Blattrührer-Apparatur in 1000 ml Phosphatpuffer pH 6.8 mit einem Zusatz an 0.4 % Tween® 80 untersucht, und zwar bei 37° C. Die erhaltenen Ergebnisse sind in Fig. 1 gezeigt.

## Patentansprüche

1. Feste Darreichungsform, die eine Matrix mit einer oder mehreren stark lipophilen physiologisch aktiven Substanzen, einem oder mehreren wasserlöslichen Bindemitteln und nicht mehr als 20 Gew.-%, bezogen auf das Gewicht aller Komponenten, an weiteren Hilfsstoffen aufweist, wobei eine physiologisch aktive Substanz stark lipophil ist, wenn sie einen log P von 4 oder mehr hat.

2. Darreichungsform nach Anspruch 1, wobei als stark lipophile physiologisch aktive Substanzen ein oder mehrere stark lipophile pharmazeutische Wirkstoffe enthalten sind.

3. Darreichungsform nach Anspruch 2, wobei als stark lipophile Wirkstoffe ein oder mehrere Cannabinoide enthalten sind.

4. Darreichungsform nach Anspruch 3, wobei ein oder mehrere Cannabinoide enthalten sind, die ausgewählt sind aus Verbindungen einer der folgenden allgemeinen Formeln (1), (3) oder (4): 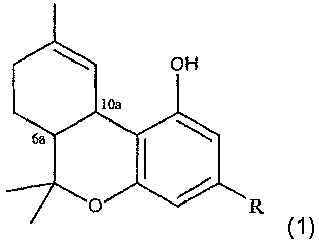 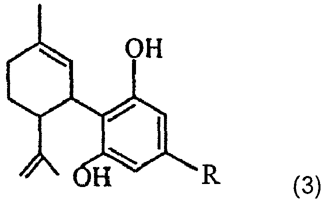 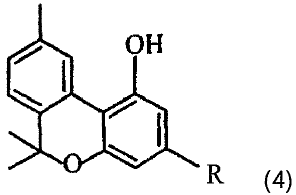
worin R jeweils ausgewählt ist aus C₁-C₂₀-Alkyl, C₂-C₂₀-Alkenyl oder C₂-C₂₀-Alkinyl und optional einen oder mehrere Substituenten aufweist;
Cannabinoiden oder Cannabinoid-Gemischen aus Hanfextrakten, wie Nabiximols; und
synthetischen Cannabinoiden, wie Nabilon oder 1-Naphthyl-(1-pentylindol-3-yl)methanon.

5. Darreichungsform nach Anspruch 4, wobei es sich bei dem Cannabinoid um 2-[1R-3-Methyl-6R-(1-methylethenyl)-2-cyclohexen-1-yl]-5-pentyl-1,3-benzenediol; (6aR,10aR)-6,6,9-Trimethyl-3-pentyl-6a,7,8,10a-tetrahydro-6H-benzo[c]chromen-1-ol oder ein Gemisch davon handelt.

6. Darreichungsform nach einem der vorstehenden Ansprüche, wobei als wasserlösliches Bindemittel Hydroxypropylmethylcellulose (HPMC) eingesetzt wird.

7. Darreichungsform nach Anspruch 6, wobei die HPMC eine Viskosität von 6 mPa·s oder weniger in einer 2% (Gew./Gew.) wässrigen Lösung bei 20°C aufweist.

8. Darreichungsform nach einem der vorstehenden Ansprüche, wobei das Bindemittel/die Bindemittel, bezogen auf die Gesamtmenge an stark lipophilen physiologisch aktiven Substanzen, in einem Gesamtanteil von 0,3 - 10 Gew.-%, vorzugsweise in einem Gesamtanteil von 0,5 - 8 Gew.-% und insbesondere in einem Gesamtanteil von 1 - 6 Gew.-%, enthalten ist/sind.

9. Darreichungsform nach einem der vorstehenden Ansprüche, die in Form eines Granulats, von Matrixpellets oder von Matrixtabletten bereitgestellt wird oder eine dieser Formen enthält.

10. Darreichungsform nach Anspruch 9, wobei die Darreichungsform Matrixpellets enthält.

11. Darreichungsform nach Anspruch 10, wobei die Matrixpellets eine Größe im Bereich von 30 bis 1800 µm, bestimmt durch Siebanalyse, aufweisen.

12. Darreichungsform nach einem der vorstehenden Ansprüche, die mehr als 30 Gew.-% und weniger als 80 Gew.-% der enthaltenen physiologisch aktiven Substanz/der enthaltenen physiologisch aktiven Substanzen innerhalb von zwei Stunden freisetzen.

13. Darreichungsform nach Anspruch 12, die mehr als 40 Gew.-% und weniger als 90 Gew.-% der enthaltenen physiologisch aktiven Substanz/der enthaltenen physiologisch aktiven Substanzen innerhalb von drei Stunden freisetzen.

14. Darreichungsform nach Anspruch 13, die mehr als 50 Gew.-% und weniger als 95 Gew.-% der enthaltenen physiologisch aktiven Substanz/der enthaltenen physiologisch aktiven Substanzen innerhalb von vier Stunden freisetzen.
